# EUROPEAN PATENT APPLICATION

(11) **EP 4 260 810 A1**
(43) Date of publication of application: **18.10.2023**
(21) Application number: 22178429.1
(22) Date of filing: 10.06.2022
(51) Int. Cl.: A61B 7/00, A61B 5/00, A61M 16/06, A62B 18/00

(54) **EXTRACTING BREATHING FEATURES WITH A FACE MASK**

(30) Priority: 14.04.2022 WO PCT/CN2022/086821
(71) Applicant: Koninklijke Philips N.V., 5656 AG Eindhoven (NL)
(72) Inventor: Zhang, Guanqun, Eindhoven (NL); Kong, Tao, Eindhoven (NL); CHEN, Weizhong, 5656AG Eindhoven (NL)
(74) Representative: Philips Intellectual Property & Standards

(57) **Abstract**

A system comprising a face mask and a processor. The face mask has a fan for ventilation and an acoustic sensor for detecting acoustic signals from within the face mask. The processor filters out the fan noise from the acoustic signals and extracts breathing features from the filtered signals.

## Description

### FIELD OF THE INVENTION

The invention relates to the field of extracting breathing features.

### BACKGROUND OF THE INVENTION

Breathing sounds contain valuable information for the detection of lung conditions. Respiratory disorders, such as chronic obstructive pulmonary disease (COPD), asthma or pneumonia, and other breathing difficulties may lead to abnormal breathing sounds.

Thus, approaches for identifying abnormal breathing sounds are of great significance. With such approaches, users could be reminded to pay attention to the condition of their respiratory system at an early stage of potential complications.

Previous studies have investigated the feasibility of abnormal respiratory sound analysis. An acoustic sensor can be placed around the thorax, neck, or mouth for collecting the breathing sound. Algorithms have been developed to detect if the abnormal sound exists or to recognize what kind of diseases the abnormal sound may relate to. However, existing solutions often require manual operation of the sensors and, thus, they are not convenient to use.

Meanwhile, face masks have become a daily necessity since the emergence of the latest coronavirus disease, COVID-19, epidemic. A face mask is a wearable and portable product which is close to the mouth. As such, the face mask may become a favorable solution for sensing breathing sound variations. However, existing face masks equipped with acoustic sensors are instead for example used to make the wearer's speech easier to hear. Furthermore, ventilation fan noise can be heard for some face masks with active fans.

It thus remains a challenge to detect abnormal breathing using a face mask.

### SUMMARY OF THE INVENTION

The invention is defined by the claims.

According to examples in accordance with an aspect of the invention, there is provided a system comprising:
a face mask comprising:
   a housing for forming a chamber over the mouth and nose of a user;
   an acoustic sensor for obtaining acoustic signals capturing breathing sounds of a user during use of the face mask; and
   a fan drawing air into or out of the chamber in use; and
a processor configured to:
   obtain a fan noise associated with the sound made by the fan when actuated;
   process the acoustic signals to filter out the fan noise; and
   extract breathing features from the processed acoustic signals.

The processor may be further configured to detect abnormal breathing features from the breathing features.

Fans facilitate the ventilation of air in the face mask therefore making the mask more comfortable during long periods of time by reducing the temperature and the moisture inside the face mask. The fan is for example for facilitating the ventilation of air exhaled by the user when the face mask is worn by the user. It may be turned on constantly or it may be operated cyclically for example synchronized with the breathing of the user.

At face value, it would seem like there is no reason to include a fan in the face mask as this would add a significant amount of noise generated by the fan to the acoustic signals, thereby significantly reducing the quality and hindering the interpretation of the acoustic signals for extracting breathing features.

However, it has been realized that the fan increases the comfort of the mask, thereby making is less likely that the user will take the face mask off due to discomfort. As such, the longer the user wears the mask, the more breathing sounds from the user which will be analyzed.

Thus, it is proposed to include a fan on the mask and remove the fan noise from the acoustic signals prior to extracting the breathing features.

The face mask may further comprise a communications module (e.g. Bluetooth module) configured to communicate with the processor via a wireless connection (e.g. Bluetooth). The processor may be an external processor forming part of an external device (e.g. mobile phone) also comprising a communications module. Alternatively, the processor is contained within the face mask.

The processed acoustic signals may be referred to as filtered signals.

The processor may be further configured to obtain calibration acoustic signals, by obtaining acoustic signals at a first time when the user is breathing, and extract the breathing features from the acoustic signals, obtained at a second later time, by comparing the acoustic signals to the calibration acoustic signals.

The calibration acoustic signals contain typical breathing features of the user (e.g. breathing pattern, minimum/maximum intensity values etc.) and the calibration acoustic signals enable calibration of the acoustic properties of the mask.

Current breathing features can be extracted by comparing the acoustic signals with the calibrated acoustic signals. The fan is switched off when measuring the calibration acoustic signals.

The processor may be configured to obtain the fan noise by obtaining acoustic signals when the user is not breathing and the fan is actuated.

Alternatively, the processor may obtain the fan noise from previous measurements of the fan noise and/or based on known spectral signatures of the particular fan used.

Knowing or measuring the fan noise associated with the fan enables the processor to remove the noise of the fan from the acoustic signals without significantly affecting the other sounds in the acoustic signals.

The system may further comprise a fan controller for actuating the fan and controlling the speed of the fan. The processor may be configured to obtain a plurality of fan noise measurements by obtaining acoustic signals when the user is not breathing, such that, for each fan noise measurement, the fan controller controls the fan at a different of fan speed and filter out the fan noise associated with the current fan speed from the acoustic signals.

Fan noise typically changes depending on the fan speed. In general, the higher the fan speed, the higher pitched the noise of the fan. The particular pitches and pitch increase/change will depend on the type of fan used.

Thus, it is proposed to obtain fan noise measurements corresponding to different fan speeds for the particular fan used such that the fan noise removed from the acoustic signals correspond to the current fan speed. This will reduce the amount of residual fan noise in the acoustic signal after the removal of the fan noise measurements.

The system may further comprise a temperature sensor for obtaining the body temperature of the user.

The temperature sensor may be detachably connected to the mask via a cable.

The temperature sensor may be a temporal artery temperature sensor or a tympanic temperature sensor.

The face mask may comprise two straps for placing around the ears of the user, thereby to hold the face mask around the face of the user. At least one temperature sensor may be positioned on at least one of the straps to measure the temperature of the superficial temporal artery of the user when the user is wearing the mask.

The straps of face masks are typically used around the ears of the user to hold the face mask in place. It has been noticed that these straps cross the superficial temporal artery of the user. Thus, temperature sensors can be advantageously positioned on the straps of the face mask, where the straps cross the superficial temporal artery, to measure the temperature of the superficial temporal artery.

The processor may be further configured to obtain a correlation function for correlating the temperatures of a superficial temporal artery obtained from the skin surface to the body temperature of the user and apply the correlation function to the temperature obtained from the temperature sensor thereby to obtain the body temperature of the user.

It has been found that the temperature of the superficial temporal artery, as measured from the surface of the skin of the user, is typically lower than the real body temperature of the user.

A correlation function can be determined relative to temperature measurements of the frontal branch of the superficial temporal artery using a typical temporal artery thermometer. The correlation function is generally linear for the range of body temperatures typically observed and depends on the position along the length of the ear.

The processor may be further configured to determine the likelihood of one or more respiratory conditions based on the abnormal breathing features and the body temperature of the user.

The processor may be configured to determine the likelihood of pneumonia based on the abnormal breathing features and the body temperature of the user.

The invention also provides a computer-implemented method for extracting breathing features from acoustic signals corresponding to a user wearing a face mask with a fan, the method comprising:
obtaining acoustic signals capturing breathing sounds of a user during use of the face mask;
obtaining a fan noise associated with the sound made by a fan when actuated;
processing the acoustic signals to filter out the fan noise; and
extracting breathing features from the acoustic signals.

The method may further comprise detecting abnormal breathing features from the breathing features.

The method may further comprise obtaining calibration acoustic signals by obtaining acoustic signals at a first time when the user is breathing and extracting the breathing features from the acoustic signals, obtained at a second later time, by comparing the acoustic signals to the calibration acoustic signals.

Obtaining a fan noise may comprise obtaining acoustic signals when the user is not breathing and the fan is actuated.

The method may further comprise obtaining a temperature of the superficial temporal artery of the user from the skin surface and applying a correlation function to the obtained temperature of the superficial temporal artery thereby to obtain the body temperature of the user.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a method for detecting abnormal breathing using a face mask with a fan;
Fig. 2 shows a schematic diagram of a system with a face mask and an external smart device;
Fig. 3 shows a method for filtering the acoustic signal;
Fig. 4 shows a face mask with a temperature sensor;
Fig. 5 shows a face mask with a temperature sensor for measuring the temperature of the frontal branch of the superficial temporal artery of a user;
Fig. 6 shows a face mask with a temperature sensor for measuring the tympanic temperature of a user;
Fig. 7 shows a face mask comprising two temperature sensor for measuring the temperature of the superficial temporal artery of a user;
Fig. 8 shows four separate points on the skin surface near an ear corresponding to the superficial temporal artery;
Fig. 9 shows a graph of the data pairs corresponding to point T1 as shown in Fig. 8;
Fig. 10 shows a graph of the data pairs corresponding to point T2 as shown in Fig. 8;
Fig. 11 shows a graph of the data pairs corresponding to point T3 as shown in Fig. 8; and
Fig. 12 shows a graph of the data pairs corresponding to point T4 as shown in Fig. 8.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a face mask with a fan to address the problem of extracting breathing features. The face mask has an acoustic sensor for detecting acoustic signals from which a breathing features can be extracted. The face mask has a processor which can filter out the fan noise from the acoustic signals. Additionally, the fan noise may be filtered out adaptively according to the fan speed, thereby improving the accuracy of breathing sound detection. The breathing features enable abnormal breathing features to be detected.

The use of face masks has become ubiquitous all over the world since the outbreak of COVID-19. Thus, placing an acoustic sensor in a face mask may enable consistent breathing sound analysis and the recognition of abnormal breathing.

Face masks are often equipped with an active electric fan for facilitating the ventilation of exhaled air. The fan improves the user comfort by reducing both temperature and humidity inside the face mask. However, the fan also generates unwanted fan noise as the fan rotates. Thus, it would be difficult to measure the breathing sounds of the user clearly when the fan is actuated.

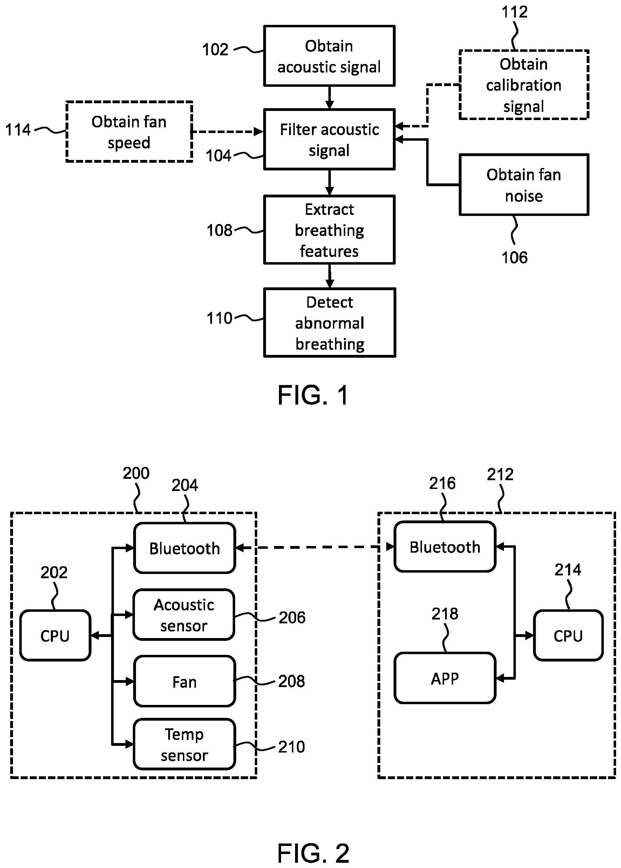

Fig. 1 shows a method for detecting abnormal breathing using a face mask with a fan. At a first, earlier time, the method may comprise calibrating the face mask with user's breathing sounds and the fan noise.

For example, at a first time of use, the user could calibrate the acoustic properties of the face mask. The user could breathe at a normal rate (i.e. at rest) for a certain period of time (e.g. 2 minutes) to obtain calibration signals in step 112 which include an ambient noise and breathing patterns of the user.

The fan could then be switched on and the user is instructed to hold their breath for a second period of time (e.g. 30 seconds). The fan noise could thus be obtained when the user is not breathing in step 106. Fans rotating at a fixed speed typically have a consistent sound spectrum over time. The particular sound spectrum may depend on the environment in which the fan is actuated. In this case, the fan is actuated within a chamber formed between the user's face and the housing of the face mask, which may have a different sound spectrum as the same fan when actuated in free space. Thus, obtaining the fan noise within the chamber provides an accurate fan noise for the given environment.

A fan controller could be used to switch the fan speed of the fan from the slowest speed to the fastest speed whilst the user is holding their breath in order to obtain various fan noise profiles corresponding to different fan speeds.

The calibration may begin by, for example, the user clicking a button on the face mask or opening a breathing/mask APP. The acoustic sensor would thus begin collecting the calibration signal including sound and noise information. The calibration signal includes the user respiration pattern and ambient noise detected from inside and outside the face mask (but measured from the inside of the mask).

Physiological parameters, including breathing rate, rhythm, and depth, may also be included in the calibration signal.

When the face mask is in daily usage, the acoustic sensor of the face mask collects the acoustic signals within the face mask continuously when it is worn by the user in step 102. The fan parameters, including the fan rotation speed, may also be simultaneously recorded in step 114.

The user may begin obtaining the acoustic signal by, for example, clicking a button on the face mask or opening a breathing/face mask APP on an external smart device to start the collection of the acoustic signal. Alternatively, the face mask may support an automatic wearing recognition function. When the fan is activated, the mask may be considered to be worn by the user. A central processing unit (CPU) on the face mask may send an instructions to the acoustic sensor to begin collecting the acoustic signal. The collected acoustic signals may be transmitted (e.g. through Bluetooth) to the breathing/face mask APP for processing and analysis.

The acoustic signals are processed by filtering out the fan noise from the acoustic signals in step 104. For example, the CPU may attenuate the noise interference caused by the fan (i.e. the fan noise) from the acoustic signal.

During acoustic signal measurements, the face mask may also record the fan rotation speed. For each level of fan rotation speed, the noise spectrum may be fixed.

The fan speed, and other hardware properties of the fan, can be obtained in step 114. This enables the spatial and/or temporal characteristics of the fan noise to be determined. For example, lower noise frequencies are expected when the fan rotates slowly while higher noise frequencies are expected as the fan rotates faster. By adaptively attenuating the specific noise interference in the acoustic signal caused by the fan motion (i.e. attenuating specific frequencies coarsening to the fan noise), the quality of the filtered signal, containing the breathing sounds without the fan noise, could be greatly enhanced.

For example, at the slower rotation mode, the fan may introduce the noise around a characteristic frequency of 7 kHz. When the fan is set as a faster rotation mode, the noise spectrum is increased up to around 10 kHz.

Sound features related to the breathing of the user (i.e. breathing features) are extracted from the sound features in step 108. The breathing features are analyzed to detect abnormal breathing in step 110. For example, a breathing/face mask APP could be used to analyze the breathing features.

Quantitative metrics may be utilized to evaluate the likelihood of abnormal breathing sound events. The metrics may include statistical computations, such as probability, correlation, cosine similarity, etc. The detection of abnormal breathing may be based on a pre-trained neural network, support vector machine, random forest, k-nearest neighbor classifier, or gradient boosting decision tree. If the breathing features are more alike to those derived from the normal breathing sounds, the received acoustic signal will be considered as normal. Otherwise, the algorithm may regard the received acoustic signal abnormal if the similarity is low in comparison with the normal breathing sound features. Furthermore, the algorithm could even compare the sound features to various known breathing features, corresponding to different diseases, to determine which kind of diseases are most probable.

Additionally, the frequency of abnormal breathing features could be measured. If the occurrence of abnormal breathing is more frequent than a pre-set threshold during a certain period (e.g. 5 times within 24 hours), a notification may be provided to the user to alert them of possible respiratory system issues. The notification could be sent to a connected external smart device or to a paired breathing/face mask application (APP).

After determining whether the breathing features indicate normal breathing or abnormal breathing, the breathing/face mask APP could export the data of the breathing features and/or the evaluation results to the user using texts, visual graphics, and/or sound indications. If abnormal breathing is detected, the external smart device could display a pop-up notification reminding the user to pay attention to their breathing health.

It is also possible to compare the breathing features (e.g. breathing features classified as abnormal breathing) with a clinical database for evaluating what kind of abnormal breathing sound it belongs to. The comparison may provide pre-clinical screening suggestions to the user.

The collected acoustic signal could by analyzed on the face mask to determine if the abnormality exists. Alternatively, the face mask could connect with an external smart device and transfer the acoustic signal to the external device using, for example, Bluetooth. The external smart device could analyze the received acoustic signal to recognize abnormal breathing.

Fig. 2 shows a schematic diagram of a system with a face mask 200 and an external smart device 212. The face mask 200 comprises an acoustic sensor 206 for collecting sound signals (i.e. acoustic signals) in the mask, a Bluetooth module 204 for communicating with the external smart device 212 (e.g. a smartphone or a smart watch). The information transferred from the face mask 200 to the device 212 may include settings of the active electric fan 208 as well as the acoustic signals collected by the acoustic sensor 206.

The computer central processing unit (CPU) 202 of the face mask 200 is for setting up the working mode of the face mask 200, storing collected acoustic signals and instructing the Bluetooth module 204 to transfer the data to the external device 212.

The active electric fan 208 improves the user comfort by reducing the temperature and humidity inside the face mask 200 through ventilation and may be controller by a fan controller (not shown). The fan controller may be part of the CPU 202 or a separate controller.

Optionally, a temperature sensor 210 could be connected to the mask via a cable and the data collected by temperature sensor 210 may be displayed on a screen or sent to and displayed on the external device 212.

The external smart device 212 has a Bluetooth module 216 for communicating with the face mask 200. It may also include a breathing APP or a face mask APP 218 for dealing with user interactions and presenting the analysis acoustic signals. Other related parts, such as the power supply of the face mask 200 or screen of the external device 212, are not shown in Fig. 2.

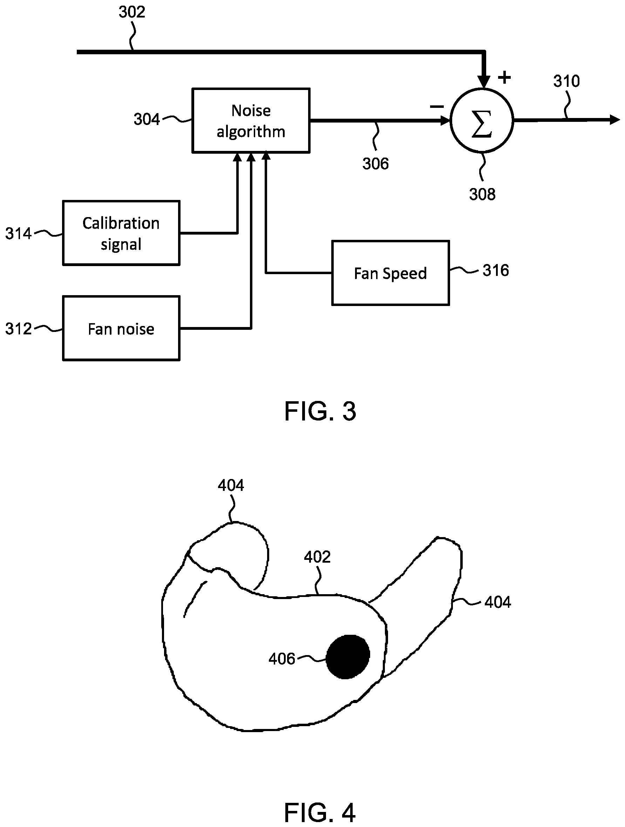

Fig. 3 shows a method for filtering the acoustic signal 302. The calibration signal 314, the fan noise 312 and the fan speed 316 are input into a noise algorithm 304. The fan noise 312 may be a set of fan noise profiles corresponding to different fan speeds. Thus, the noise algorithm 304 may use the fan noise 312 and the fan speed 316 to determine a fan noise signal 306 which is to be filtered out (i.e. removed/attenuated) from the acoustic signal 302.

For example, at the slower fan speed, the fan noise frequency may concentrate around 7 kHz for a conventional fan used in face masks. The noise frequencies may increase to around 10 kHz at a faster fan speed. Therefore, specific frequencies may be filtered out from the acoustic signal depending on the fan speed.

The calibration signal 314 may also be input into the noise algorithm 304 to include the ambient noise in the fan noise signal 306 and thereby remove the ambient noise from the acoustic signal 302. However, not all ambient noise may be able to be removed as its properties can vary over time. Only sounds with similar characteristics to the calibration signal can be attenuated. In practice, this attenuation is acceptable for daily use. If the acoustic sensor detects the ambient noise properties have significantly deviated from those at calibration, it may remind the user to recalibrate for improved performance.

A signal combiner 308 is used to filter out the fan noise profile 306 from the acoustic signal 302 and output a clean signal 310 corresponding to the breathing sounds of the user without any fan or ambient noise. The signal combiner thus performs a filtering function, typically in the frequency domain.

Signal processing techniques for filtering out the fan noise from the acoustic signal will be known. For example, an electronic circuit could be built using band-stop filters corresponding to the fan noise frequency peaks. Alternatively, other signal processing algorithms may be generated/used which attenuate or remove the frequency profile corresponding to the fan noise.

After filtering out the fan noise, the filtered signal 310 is processed for feature extraction. The breathing features extracted may consist of temporal, spatial and/or higher-order statistical domains. For example, in the temporal domain, the mean, median, maximal value, minimal value, centroid, correlation coefficient, time duration and/or slope could be determined.

In the spatial domain, the power spectrum density, wavelet coefficients, Mel frequency cepstral coefficients and/or an empirical mode decomposition could be determined.

In the higher-order statistical domain, entropy-based features, skewness, kurtosis, fractal dimension, and/or autoregressive model coefficients could be determined.

The particular breathing features which are extracted from the acoustic signal 302 may depend on the available processing resources, the particular diseases which the user/programmer may want to detect or known features for detecting abnormal breathing.

Both the noise filtering and feature extraction can be performed on the face mask directly or processed in the external smart device after the face mask sends the signals to the external device.

In summary, a system is provided comprising a face mask and a processor. The face mask has a fan for ventilation and an acoustic sensor for detecting acoustic signals from within the face mask. The processor filters out the fan noise from the acoustic signals and detects abnormal breathing from the filtered signals.

Fig. 4 shows a face mask with a temperature sensor 406. The face mask comprises a housing 402 which form a chamber with the face of the user when the user wears the mask and two straps 402 for placing around the ears of the user to hold the face mask against the face of the user.

Body temperature measurement may be provided by the face mask. In order to implement body temperature measurement in a face mask, four non-invasive body temperature measurement methods have been identified: axillary, oral, tympanic and frontal branch of the superficial temporal artery temperature measurements.

Axillary temperature measurements are generally not feasible or practical for utilization on a face mask due to the distance between the face mask and the underarm of the user. Oral temperature methods may lead to sanitary issues in face mask scenarios. However, tympanic and superficial temporal artery temperature measurements have been found to be feasible and practical in the case of face masks.

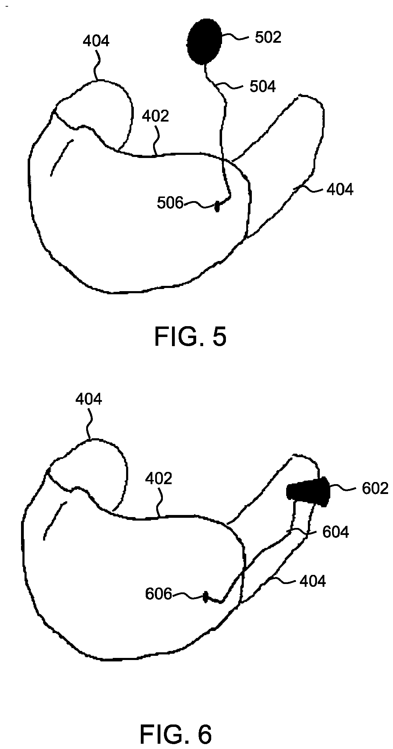

Fig. 5 shows a face mask with a temperature sensor 502 for measuring the temperature of the frontal branch of the superficial temporal artery of a user.

The temporal artery temperature sensor 502 measures the body temperature by detecting the heat radiated from the frontal branch of the superficial temporal artery. The superficial temporal artery is a branch of the external carotid artery which is directly derived from heart such that the temperature of this artery is very close to the core body temperature. The frontal branch of the superficial temporal artery lays very superficial under the skin of the forehead and thus makes it the most suitable location on the face for direct body temperature measurement.

The superficial temporal artery temperature sensor 502 is connected to the face mask via a cable 504 which provides power to the temperature sensor 502, enables the transfer of data from the temperature sensor 502 to the face mask and conveniently attaches the temperature sensor 502 to the housing 402 of the face mask. After the temperature measurements, the temperature sensor 502 may be re-attached to the mask at point 506 on the housing 402 for conveniently holding the temperature sensor 502 whilst it is not in use. The temperature sensor 502 and the cable 504 could also be designed as a separated attachment of face mask and could be connected to the face mask via a plug at the end of the cable 504 which is plugged into point 506 of the housing 402.

As such, the temperature sensor 502 may be attached to the face mask when it is not in use or the sensor could be unplugged from the face mask when not in use.

Fig. 6 shows a face mask with a temperature sensor 602 for measuring the tympanic temperature of a user. Tympanic thermometers, such as temperature sensor 602, typically use infrared rays to measure the temperature of the eardrum, which is inside the ear canal. Similarly to temporal artery temperature sensor 502 shown in Fig. 5, the tympanic temperature sensor 602 is connected to the face mask via a cable and may be disconnected from the face mask when not in use.

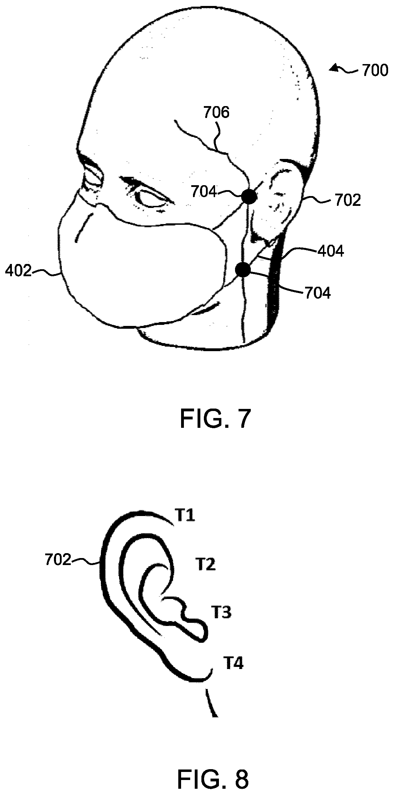

Fig. 7 shows a face mask comprising two temperature sensors 704 for measuring the temperature of the superficial temporal artery 706 of a user 700. Typically, the temperature of the superficial temporal artery 706 is measured at the frontal branch near the forehead as that is the place at which the temperature measurements most closely match the real body temperature due the temporal artery being closer to the skin. However, it has been realized that during normal use of a face mask, the straps 404 of the face mask cross the superficial temporal artery 706 near the ear 702 of the user 700, thus making it a convenient spot for placing temperature sensors 704.

The temperature sensors 704 may be attached to the skin area above the temporal artery, connected and powered by a mask battery, and also attached onto the straps 704 of the face mask. Body temperature can thus be obtained by modelling a correlation function between the skin surface temperature measured by the temperature sensors 704 and the real body temperature.

The implementation of the temperature sensors 704 on the straps 404 provides a convenient and non-obstructive method for measuring the body temperature of a user wearing a mask, thus enabling a comfortable, visually imperceptible and continuous monitoring the body temperature of the user 700 when the face mask is worn.

Continuous body temperature measurements may be relevant for monitoring a child's body temperature when they have a fever. Additionally, for women who are preparing for pregnancy, changes in body temperature could indicate the date of ovulation.

The temperature sensors 702 may be moveable along the straps 404 such that they can be positioned according the particular user 700. An algorithm may be provided which informs the user where to place the temperature sensors along the strap to ensure correct placement on the superficial temporal artery.

Fig. 8 shows four separate points on the skin surface near an ear 702 corresponding to the superficial temporal artery. Point T1 often corresponds to the placement of the top of the strap of a face mask whilst point T4 often corresponds to the placement of the bottom of the strap. Correlation functions have been determined for each of the four point T1, T2, T3 and T4. The correlation functions were determined by measuring 15 data pairs between true body temperature, using a superficial temporal artery thermometer at the frontal branch, and the temporal artery surface skin temperature at points T1, T2, T3 and T4. Linear correlations were found between the temporal artery surface skin temperatures at T1, T2, T3 and T4 and true body temperature for a range of expected body temperatures between 36.5 degrees and 38 degrees Celsius. All temperatures below are in degrees Celsius.

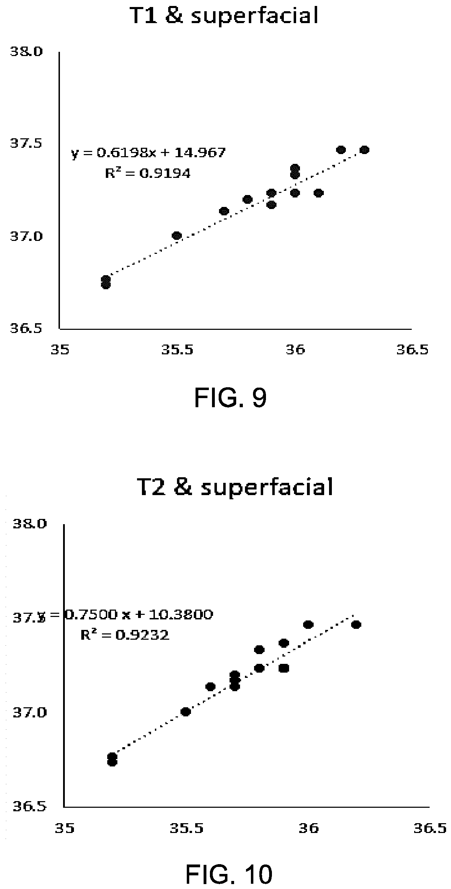

Fig. 9 shows a graph of the data pairs corresponding to point T1 as shown in Fig. 8. The y axis shows the real body temperature and the x axis shows the temperature measurements at point T1. The correlation function for point T1 was determined to be y = 0.6198x + 14.967 with an R² = 0.9194.

Fig. 10 shows a graph of the data pairs corresponding to point T2 as shown in Fig. 8. The y axis shows the real body temperature and the x axis shows the temperature measurements at point T2. The correlation function for point T2 was determined to be 0.7500x + 10.3800 with an *R*² = 0.9232.

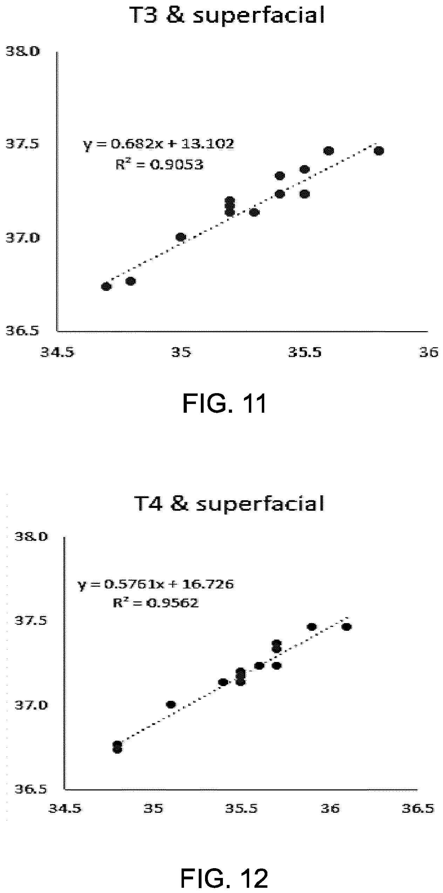

Fig. 11 shows a graph of the data pairs corresponding to point T3 as shown in Fig. 8. The y axis shows the real body temperature and the x axis shows the temperature measurements at point T3. The correlation function for point T3 was determined to be 0.682x + 13.102 with an *R*² = 0.9053.

Fig. 12 shows a graph of the data pairs corresponding to point T4 as shown in Fig. 8. The y axis shows the real body temperature and the x axis shows the temperature measurements at point T4. The correlation function for point T4 was determined to be 0.5761x + 16.726 with an *R*² = 0.9562.

Using the correlation functions obtained above, the true body temperature can be calculated from the temporal artery surface skin temperatures at points T1, T2, T3 and T4. Different correlation functions for points other than T1, T2, T3 or T4 may need to be obtained if the particular straps of a face mask cross the temporal artery at a different position. In some cases, the correlation function could be personalized to a particular user if the real body temperature of the user can be found.

The correlations functions found in Figs. 9 to 12 were tested against real temperature measurements at a different day. The predicted real body temperatures obtained from the correlation functions showed that the accuracy of this method is between (-0.2, 0.4) degrees Celsius. This error is generally acceptable for daily use.

Regardless of the method for obtaining the body temperature, the body temperature may be displayed on a screen (e.g. on the temperature sensor or on the face mask) or may be displayed on a connected external device (e.g. a smart phone or smart watch).

Body temperature may be relevant for evaluating the abnormalities in the respiratory system. Thus, the face mask may be able to obtain the body temperature of the user and acoustic signals from within the face mask, both of which are important indicators of respiratory conditions. Although each factor (i.e. body temperature and acoustic signal) is clinically relevant to respiratory diseases, such as pneumonia, the combination of the two may enhance the detection rate significantly. For example, Hopstaken et al. proposed a formula for estimating the pneumonia probability when fever and/or dry cough exists.

Thus, the combination of acoustic sensors and temperature sensors on a face mask may enable an accurate estimation of the probability of respiratory system abnormalities. If the probability exceeds a pre-defined threshold (e.g. > 60%), a warning may be sent to the user automatically.

The temperature sensors could also be used to indicate the temperature within the mask and thus inform the fan speed (i.e. higher fan speeds at higher temperatures to increase comfort). The fan noise will then also be automatically correctly filtered out when fan speed adjustments are made in response to the sensed temperature.

The skilled person would be readily capable of developing a processor for carrying out any herein described method. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A system comprising:
a face mask (200) comprising:
a housing (402) for forming a chamber over the mouth and nose of a user;
an acoustic sensor (206) for obtaining (102) acoustic signals capturing breathing sounds of a user during use of the face mask; and
a fan (208) drawing air into or out of the chamber in use; and
a processor (202) configured to:
obtain (106) a fan noise associated with the sound made by the fan when actuated;
process (104) the acoustic signals to filter out the fan noise; and
extract (108) breathing features from the processed acoustic signals.

2. The system of claim 1, wherein the processor is further configured to detect (110) abnormal breathing features from the breathing features.

3. The system of claims 1 or 2, wherein:
the processor is further configured to obtain (112) calibration acoustic signals by obtaining acoustic signals at a first time when the user is breathing, and
the processor is configured to extract the breathing features from the acoustic signals, obtained at a second later time, by comparing the acoustic signals to the calibration acoustic signals.

4. The system of any one of claims 1 to 3, wherein the processor is configured to obtain the fan noise by obtaining acoustic signals when the user is not breathing and the fan is actuated.

5. The system of claim 4, comprising a fan controller for actuating the fan and controlling the speed of the fan, wherein:
the processor is configured to obtain a plurality of fan noise measurements by obtaining acoustic signals when the user is not breathing, such that, for each fan noise measurement, the fan controller controls the fan at a different of fan speed; and
the processor is configured to filter out the fan noise associated with the current fan speed from the acoustic signals.

6. The system of any one of claims 1 to 5, further comprising a temperature sensor (210, 406) for obtaining the body temperature of the user.

7. The system of claim 6, wherein the temperature sensor is a temporal artery temperature sensor (502) or a tympanic temperature sensor (602).

8. The system of claim 7, wherein:
the face mask comprises two straps (404) for placing around the ears of the user, thereby to hold the face mask around the face of the user, and
at least one temperature sensor is positioned on at least one of the straps to measure the temperature of the superficial temporal artery of the user when the user is wearing the mask.

9. The system of claim 8, wherein the processor is further configured to:
obtain a correlation function for correlating the temperatures of a superficial temporal artery obtained from the skin surface to the body temperature of the user; and
apply the correlation function to the temperature obtained from the temperature sensor thereby to obtain the body temperature of the user.

10. The system of any one of claims 5 to 9, wherein the processor is further configured to determine the likelihood of one or more respiratory conditions based on the breathing features and the body temperature of the user.

11. The system of claim 10, wherein the processor is configured to determine the likelihood of pneumonia based on the breathing features and the body temperature of the user.

12. A computer-implemented method for extracting breathing features from acoustic signals, the method comprising:
obtaining (102) acoustic signals capturing breathing sounds of a user during use of the face mask;
obtaining (106) a fan noise associated with the sound made by a fan when actuated;
processing (104) the acoustic signals to filter out the fan noise; and
extracting (108) breathing features from the acoustic signals.

13. The method of claim 12, further comprising detecting (110) abnormal breathing features from the breathing features.

14. The method of claims 12 or 13, further comprising:
obtaining (112) calibration acoustic signals by obtaining acoustic signals at a first time when the user is breathing, and
extracting the breathing features from the acoustic signals, obtained at a second later time, by comparing the acoustic signals to the calibration acoustic signals.

15. The method of any one of claims 12 to 14, wherein obtaining a fan noise comprises obtaining acoustic signals when the user is not breathing and the fan is actuated.
